(19)

Europäisches
Patentamt
European
Patent Office
Office européen
des brevets

(11) **EP 4 349 262 A1**

(12) **EUROPEAN PATENT APPLICATION**

(43) Date of publication:
**10.04.2024 Bulletin 2024/15**

(21) Application number: **22199939.4**

(22) Date of filing: **06.10.2022**

(51) International Patent Classification (IPC):
**A61B 6/00** (2024.01)

(52) Cooperative Patent Classification (CPC):
**A61B 6/481; A61B 6/4241; A61B 6/482;
A61B 6/504; A61B 6/5217; A61B 6/5258**

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**KH MA MD TN**

(71) Applicant: **Koninklijke Philips N.V.
5656 AG Eindhoven (NL)**

(72) Inventors:
- **HAASE, Hans Christian
  Eindhoven (NL)**
- **GRASS, Michael
  Eindhoven (NL)**

(74) Representative: **Philips Intellectual Property &
Standards
High Tech Campus 52
5656 AG Eindhoven (NL)**

(54) **FLOW STATE NORMALIZATION FOR FUNCTIONAL CTA**

(57) System and related method for medical image processing, comprising an input interface (IN) through which is receivable input data. The input data included spectral input imagery of a part of a vascular system (VS) of a patient (PAT). The spectral input imagery is based on data reconstructable from projection data acquired by a spectral imaging apparatus of the tomographic type, with presence of contrast agent in the part of the vascular system. A predictor module (PM) of the system is configured to predict, based on the input imagery, an increase or decrease in flow caused by presence of contrast agent. An output interface (u-OUT) provides output data indicative of the predicted flow increase or decrease.

**FIG. 3**

EP 4 349 262 A1

**Description**

FIELD OF THE INVENTION

The invention relates to a system for medical image processing, to an imaging arrangement, to related methods, to a computer program element and to a computer readable medium.

BACKGROUND OF THE INVENTION

Coronary CTA (computed tomography angiography) imaging is gaining importance as a non-invasive first line diagnostic tool for suspected coronary artery disease.
Specifically, CT perfusion or CT derived IMR assessment garnered recent interest since these approaches may support the diagnosis of microvascular dysfunction and thus address a longstanding problem.
An obstacle for these noninvasive methods is the influence of the venous contrast agent injection on coronary blood flow which is needed however to confer sufficient image contrast. Intracoronary contrast agent administrations may cause a full hyperemic state by a typical contrast agent dose. Under venous administration, the response of the coronary autoregulation is in-between the normal resting state and a full hyperemic state. The widening of vessels is thought to be an autoregulatory response caused by the interfering presence of the contrast agent material in the vessel.
Thus, any derived diagnostic measurements/quantities that relate to blood flow in the coronaries is affected by this response of the autoregulation. As the contrast agent dose and distribution varies from patient to patient, the autoregulation response varies, and thus it is hard to compare patient measurements directly or to derive global threshold values across patient cohorts. Specifically, CTA derived hemodynamic measurements that relate to blood flow in the coronaries (e.g. perfusion, CFR, IMR) are subject to inter- and intra- patient variability since they are affected by the response of the coronary autoregulation to contrast agent. Comparability of these measurements is thus limited.

SUMMARY OF THE INVENTION

There may therefore be a need for improved image-based measurement of flow related diagnostic, in particular hemodynamic, quantities.
An object of the present invention is achieved by the subject matter of the independent claims where further embodiments are incorporated in the dependent claims. It should be noted that the following described aspect of the invention equally applies to the related methods, the imaging arrangement, to the computer program element and to the computer readable medium.
According to a first aspect of the invention there is provided a system for medical image processing, comprising:

> an input interface through which is receivable, when the system is in use, input data including spectral input imagery of at least a part of a vascular system of a patient, the spectral input imagery based on data reconstructable from projection data acquired by a spectral imaging apparatus of the tomographic type, with presence of contrast agent in the part of the vascular system;
> a predictor module configured to predict, based on the input imagery, an increase or decrease in flow caused by presence of contrast agent; and
> an output interface for providing output data indicative of the predicted flow increase or decrease.

In embodiments, the system comprises an in-image measurement component configured to measure a medical quantity of interest based on the input imagery and the said output data.
In embodiments, the in-image measurement component is to combine information from the input imagery with information based on the said output data.
In embodiments, the combining by the in-image measurement component includes correcting or normalizing, based on the said output data, an initial measurement of the medical quantity of interest by the in-image assessment component based on the input imagery.
In embodiments, the medical quantity of interest includes a hemodynamic quantity.
In embodiments, the hemodynamic quantity includes any one or more of: perfusion score, [virtual] fractional flow reserve, FFR, [virtual] instantaneous flow ratio iFR, coronary flow reserve, CFR, index of microvascular resistance, IMR, and transluminal attenuation gradient, TAG.
In embodiments, the input data further includes one or more vital sign measurements in relation to the patient.
In embodiments, the system includes a renderer configured to provide a sensory indication based on the output data for consumption by a user.
In embodiments, the renderer includes any one or more of: i) a visualizer configured to cause displaying on a display device of the output data, ii) a transducer configured to generate an alert signal in relation to the output data.

In embodiments, the system includes a control interface configured to issue a control signal based on the output data to control medical equipment.

In embodiments, the input imagery is based on a partial reconstruction from the projection data.

In embodiments, the input imagery and/or the predicted flow increase or decrease has a time and/or location dependency.

In embodiments, the increase or decrease in blood flow is caused by an autoregulation response of the vascular system due to the presence of contrast agent.

In embodiments, the predictor component configured to correlate concentration of contract agent in the part of the vascular system, or in surrounding tissue, to said increase or decrease in contrast agent flow.

In embodiments, the predictor module is based on a trained machine learning model.

In embodiments, the spectral imaging apparatus is configured for computed tomography angiography.

In another aspect there is provided an arrangement including a system as per any one of the previous claims, and the imaging apparatus.

In another aspect there is provided a method of medical image processing, comprising:-

receiving input data including spectral input imagery of at least a part of a vascular system of a patient, the spectral input imagery based on data reconstructable from projection data acquired by a spectral imaging apparatus of the tomographic type, with presence of contrast agent in the part of the vascular system;

estimating, based on the input imagery, an increase or decrease in flow caused by presence of contrast agent; and providing output data indicative of the predicted flow increase or decrease.

In another aspect there is provided a method of training the machine leaning model based on training data.

In another aspect there is provided a computer program element, which, when being executed by at least one processing unit, is adapted to cause the processing unit to perform any one of the methods.

In another aspect there is provided at least one computer readable medium having stored thereon the program element, or having stored thereon the machine learning model.

In yet another aspect there is provide a use of the predicted flow increase in computing a diagnostic quantity, such as hemodynamic quantity. This allows improving cross-patient comparability/standardization.

What is proposed herein in embodiments is to use spectral 3D imaging, such as CTA, to estimate (implicitly or explicitly) based thereon, a response of the coronary autoregulation due to contrast agent presence. Observation of the contrast agent concentration may be used for this as captured in the CTA imagery. The response is mainly one of increased blood flow. Based on the predicted blood flow increase, the effect on diagnostic measurements may be accounted for, such as by normalization, correction, or other. In embodiments, the estimated blood flow increase may be locally or globally monitored for a given one or more regions of interest (ROI)s. Thus, an impact of the coronary autoregulation/flow increase on diagnostic measurement values may be estimated. The estimation of this impact may be done in a time and/or vessel location resolved manner. Flow based or related diagnostic measurements can be made more accurately, reliably and can be made comparable across patient cohorts, thanks to the proposed system and method. In some alternative embodiments, flow decrease is estimated instead for some classes/types of contrast agents. However, the proposed approach is mainly envisaged to estimate flow increase as this effect appears to be more prevalent among patients.

The predictor module or prediction step may be based at least in parts on analytical modelling where amounts of contrast agent are detected, thanks to the superior accuracy of spectral imagery and are (directly) mapped to a corresponding amount of increase in flow. However, such (explicit) extraction of amount of contrast agent is not required in some machine learning based applications which may be implemented end-to-end, where the input image data is mapped to the flow increase. Hybrid-type implementations using ML and analytical approaches are also envisaged herein.

The proposed imaging may be considered functional since it is the functional impact of a certain condition, such as coronary artery disease, that is investigated, as opposed to only investigate geometric/anatomical impact. For example, instead of merely measuring a lesion diameter, or diameter reduction, it is measured or simulated how a lesion impacts the blood supply function or other physiological aspect.

Whilst the above described is focused on examination of the cardiac or other vasculatures for stenosis or other lesions, others medical applications are not excluded herein, such as examination of other vessel systems such as lymphatic, urinary or gastric tracts. Neither are there excluded herein non-medical applications of the principles described herein, such as examination of inaccessible plumbing or hydraulic systems, speleological/geological/hydrological examinations of water flow and seepage, and other flow related areas of endeavor.

"*user*" relates to a person, such as medical personnel or other, operating the imaging apparatus or overseeing the imaging procedure. In other words, the user is in general not the patient.

In general, the term "*machine learning*" includes a computerized arrangement (or module) that implements a machine learning ("ML") algorithm. Some such ML algorithms operate to adjust a machine learning model that is configured to perform ("learn") a task. Other ML operate direct on training data, not necessarily using such as model. This adjusting or updating of training data corpus is called "training". In general task performance by the ML module may improve measurably, with training experience. Training experience may include suitable training data and exposure of the model to such data. Task performance may improve the better the data represents the task to be learned. "*Training experience helps improve performance if the training data well represents a distribution of examples over which the final system performance is measured*". The performance may be measured by objective tests based on output produced by the module in response to feeding the module with test data. The performance may be defined in terms of a certain error rate to be achieved for the given test data. See for example, T. M. Mitchell, "Machine Learning", page 2, section 1.1, page 6 section 1.2.1, McGraw-Hill, 1997.

BRIEF DESCRIPTION OF THE DRAWINGS

Exemplary embodiments of the invention will now be described with reference to the following drawings, which, unless stated otherwise, are not to scale, wherein:

Fig. 1 shows an overview block diagram of a medical imaging arrangement capable of obtaining spectral image data;
Fig. 2 shows a schematic block diagram of the medical arrangement in more detail and according to one embodiment;
Fig. 3 shows a block diagram of a computerized system for computing flow increase based on reconstructed spectral image data caused by the presence of contrast agent in the imaged region;
Fig. 4 shows a block diagram of a machine learning model as per one embodiment, and as may be used in the system of any one of Figs. 1-3;
Fig. 5 shows a block diagram of a training system as per one embodiment for training a machine learning model;
Fig. 6 shows a flow chart of a method of computing flow increase caused by the presence of contrast agent in an imaged region, to so facilitate computing associated one or more medical quantities of interest; and
Fig. 7 shows a flow chart of a method of training a machine learning model.

DETAILED DESCRIPTION OF EMBODIMENTS

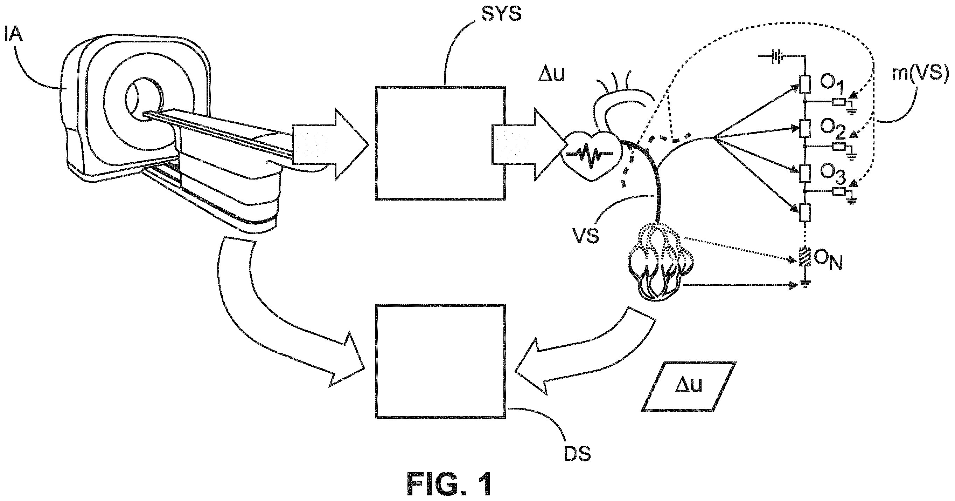

With reference to the schematic overview block diagram of Fig. 1, what is proposed herein is a computer-implemented system SYS for supporting contrast agent assisted tomographic imaging operation. The region of interest ("ROI") includes may include a vessel VS, such as a blood vessel through which blood passes. Specifically, the vessel VS may include in particular the whole or part of a cardiac vasculature in a human patient. The proposed system allows the processing volumetric contrasted image data $V=R(\lambda)$ provided by a tomographic imaging apparatus IA, operable to acquire projection imagery $\lambda$ of a region of interest (ROI), from which the volumetric imagery $V$ is reconstructable.

The preferably X-ray based imaging apparatus IA is configured for spectral imaging so that spectral volumetric image data $V=R(\lambda)$ is provided for processing by the system SYS. The spectral volumetric image data $V=R(\lambda)$ represents multiple cross-sectional (tomographic) imagery along an imaging axis, with image contrast specifically tuned, thanks to the spectral processing, to the contrast agent previously administered to the region of interest. Plural volumes may be obtained $V=V_{i\in I}$ (quasi "4D" data), with a $i$ an index in a suitable interval, such as different time instants in a time period. Some volumes in the series may be based on full scans, others on partial scans (a scan refers to the set of projection data acquired). Some angular ranges may be overlapping, others may be disjoint. Index $i$ may relate instead of, or in addition to time, to space as will be described in more detail below. The system may also take as input plural such volumes of multiple ROIs, or a single such volume with multiple ROIs.

The system SYS, as will be described below in more detail, is configured to estimate a physiological response of the vasculature due to the contrast agent being present in the vessel. In particular, and as mainly envisaged herein, the SYS, as will be described below in more detail, is configured to estimate an increase blood flow $\Delta u$ due to the contrast agent being present in the vessel. It has been found that this unwanted effect of flow increase interferes unfavorably with flow-related quantities, such as hemodynamic quantities, computable from the spectral imagery with contrast agent present, from which, in turn, other diagnostic quantities are derivable. Similarly, certain contrast agents may case decrease in blood flow. However, the below will mainly focus on flow increase estimation as mainly envisaged herein.

The flow related quantities may describe flow characteristics of the blood/contrast agent mixture. For example, such flow characterizing quantities allow building a model m(VS) of the vasculature VS of interest, including modelling of flow resistances $Oj$. An image measuring component IMC may process the imagery and the flow-related quantities, in order to compute therefrom hemodynamic quantities $d$, where the said estimated increase in flow $\Delta u$ is accounted for, such as by compensation, offsetting, normalization or other computational operation. The proposed system facilities obtaining flow-related quantities, such as hemodynamic quantities $d$, of higher fidelity, better cross-patient comparability and better accuracy. Better diagnostic results are promoted by the proposed system SYS. The hemodynamic values $d$

may be processed by a diagnostic system DS for example to compute derived quantities such as scores for the presence of a condition, etc.

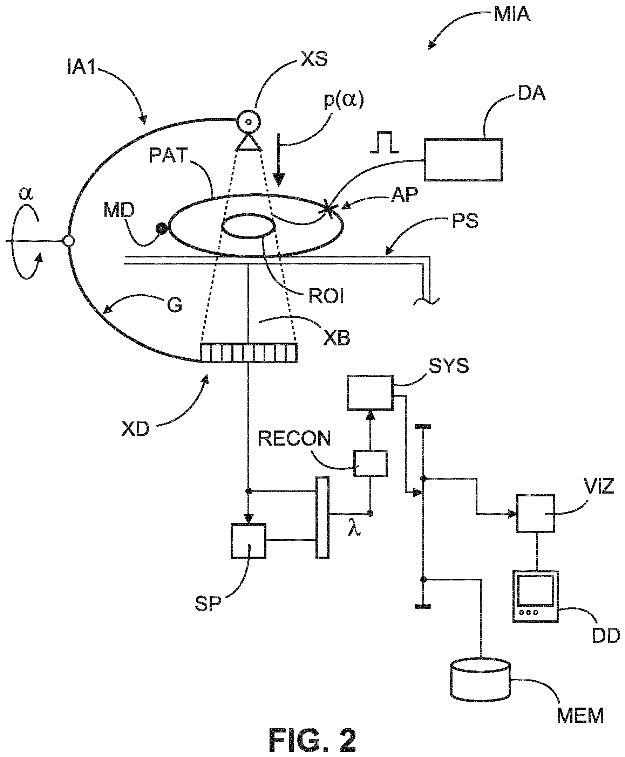

Before explaining operation of flow increase estimator/predictor system SYS in more detail, components of the overall imaging arrangement MIA are now described first, with reference to the block diagram of Fig. 2.

The medical imaging arrangement MIA may include a, preferably, X-ray based imaging apparatus IA (also referred to herein simply as "imager"). The imaging apparatus IA may be of the rotational type. Such a rotational imaging apparatus allows collection of projection imagery $\lambda$ along different projection directions $P(\alpha)$ in a "scan" around the region of interest, such as around the mentioned stricture in a coronary vessel. The imaging apparatus IA includes an X-ray source XS and an X-ray sensitive detector XD. In some embodiments, the X-ray source XS, and opposite thereto, the X-ray sensitive detector XD, are arranged preferably on a rotatable gantry G. The gantry G may have a recess in which an examination region is defined where the patient, or in particular the region of interest, resides during imaging. The rotatable gantry may thus have a "C", "U" or similar shape, such as in C-arm imaging apparatus envisaged herein. A bi-planar imaging setup with two detectors with intersecting imaging axes (for example at right angles) is not excluded herein, but is less preferred. Instead of using a C-arm/U-arm system, a CT scanner is also envisaged in some embodiments, of any generation, but is preferably of at least 3rd generation, where x-ray source XS and detector D are mounted so that they rotate together around the patient. However, for multi-directional projection data acquisition, yet higher generation CT setups are also envisaged, where there is not necessarily mechanical rotation of source and detector. Instead, a stationary ring of detectors around the ROI is used and/or stationary multiple X-sources arranged in a ring around ROI. Cone beam imaging or other divergent beam geometries are also envisaged herein, and so is imaging with a helical scan path caused by circular path of the X-ray source for example, with translational motion relative to imaging apparatus IA and ROI along patient's longitudinal axis. In most cases, this translational motion is caused by a patient bed, with patient on it, being translated during projection data acquisition. However, scan paths with geometry other than circular/helical are not excluded herein.

In addition, whilst digital-at-source imaging, such as flat panel detectors is preferred, where the imagery is natively acquired as digital data, such as setup is not necessarily required. More traditional, cassette-operable, analog imaging methods (based on X-ray films) are not excluded herein. The analog imagery may be digitized post-acquisition for example, by using a digital camera. Imaging based on X-ray image intensifiers, etc with post-digitization is also envisaged in some embodiments.

Generally, imaging includes energizing the X-ray source XS, so that an X-ray beam XB issues forth from a focal spot of the source XS, traverses the examination region (with the region of interest in it) and interacts with patient tissue matter. Interaction of X-ray beam XB with tissue matter causes the beam to be modified. The modified beam may then be detected at the detector XD. Conversion circuitry (not shown) converts detected intensities into digital imagery, in particular into projection imagery. The imagery may be used by user in the medical procedure. The patient may reside on a patient support PS such as a table during imaging. Soft tissue such as the one making up the coronary vessels, provide usually poor contrast in native X-ray imagery.

In order to boost contrast, a contrast agent (sometimes "CA" or short), such as an iodine solution, gadolinium, or other, is delivered through an access point AP into the blood flow of the patient prior to or during imaging. The contrast agent may be delivered via a CA delivery apparatus DA, such as a motorized pump, but manual delivery is not excluded herein. The delivery apparatus DA may be operable to deliver a defined volume of contrast agent to the patient. The volume of contrast agent so delivered travels with the blood flow. After a certain period of time contrast agent accumulates at the region of interest such as in or around the stricture. Image acquisition is preferably synchronized with arrival of contrast agent at the ROI. Preferably "contrasted" imagery is acquired whilst there is contrast agent present at the ROI. Optionally, additional "non-contrasted" imagery is acquired whilst no contrast agent is present at the ROI.

The in particular contrasted projection imagery may be acquired in a time series with varying concentration of the contrast agent accumulating at the region of interest. Image acquisition may start before, may continue throughout, and may terminate after accumulation of the contrast agent at the ROI. In general, contrast agent concentration increases over time after delivery, will plateau at a certain maximum value, and will then wash out or decrease over time until completely vanished. Whilst a single or certain discrete number of still images may be acquired in some instances, acquisition of a stream images, or frames, acquired at a suitable frame rate is preferred as this allows real time dynamic observation. Specifically, the acquired stream of projection imagery may be visualized by a visualizer VIZ as a live video feed on display device DD. Alternatively or in addition, still imagery is so visualized. Operation of the proposed system SYS is based on processing such still or stream imagery. The imagery may be stored in an image memory MEM or may be processed otherwise.

X-ray imaging is generally based on the attenuation co-efficient of the matter(s) that makes up the region of interest. The attenuation (coefficient) is dependent on the energy of the X-ray beam. In order to harness this energy dependency, a spectral imager IA may be used, configured for spectral imaging. This may include detector-sided or source-sided solutions. Detector-sided solutions include photon-counting detector hardware or a dual-energy setup with dual-layer hardware for example. More than two detector layers may be used although this is less common. Source-side solutions include multi-source setups, kVp switching, filtration and other.

The imaging set-up in spectral imaging allows acquisition of multi-dimensional image data. Such multi-dimensional image data includes projection imagery at different energy levels, instead of acquiring energy integrated imagery as provided by a conventional (non-spectral) X-ray imaging set-up, and along different projection directions $\alpha$.

Such a spectral CT imaging setup includes a spectral image processor SP. The spectral image processor SP may implement a spectral image processing algorithm, such as material decomposition or other. A range of such spectral image processing algorithms are envisaged herein. The spectral processor SP processes the multi-energy projection imagery $\lambda$ acquired by the source- or detector-sided imaging set-up just described, and computes therefrom spectral imagery that differs from the original input multi-energy projection imagery. Spectral projection imagery is thus computationally derived from the input multi-energy projection imagery.

Such spectral imagery, which may be in projection domain or image domain, may include in particular a contrast-only-image ("VC") as mainly envisaged herein. Such a VC image represents an approximation of an image that may have been obtained if there had been only contrast agent present in the field of view of the imager IA, at the exclusion of other materials. Thus, image contrast in VC is exclusively or pre-dominantly contrast agent focused.

A tomographic reconstructor RECON processes projection data into tomographic/volumetric imagery $R(\lambda)=V$ as will be described in more detail below. The volumetric data V is spectral image data due to the spectral processor SP. The spectral processor SP may operate in projection domain on projection data $\lambda$ as acquired, or may operate in image domain after reconstruction. For simplicity, the notation "$V$" or "$R(\lambda)$" may be used herein to indicate spectral volumetric imagery in image domain, irrespective of the domain in which the spectral processing SP took place. It is such spectral volumetric data V that is processed by the proposed system SYS. The spectral processor SP may be integrated into the reconstructor RECON.

The spectral processor SP and the proposed system SYS may be implemented on different computing systems, possibly geographically separated such as in a distributed "cloud" architecture. However, implementation on a single computing system is also envisaged herein. Imaging as envisaged herein is preferably attenuation-based, but this is not at the exclusion of other X-ray modalities, such as dark-field imaging and/or phase contrast imaging.

The reconstructor RECON is configured to reconstruct the volumetric imagery V= $R(\lambda)$ from the acquired projection data $\lambda$, the said projection data $\lambda$ acquired whilst at least some contrast agent is present in the field of view of the imager IA, and thus in region of interest including at least a part of a vasculature of interest.

The system SYS is mainly envisaged herein to operate on spectral image volumetric data R($\lambda$). However, some embodiments of system SYS operable in projection domain itself is not excluded herein, although this is less preferred. Projection domain is the space situated at the detector where the projection data is detected. Tomographic reconstruction is implemented by reconstructor RECON is a transformation operation $R$ from which the volumetric data is computed. The reconstruction transformation operation $R$ is a transformation from said projection domain to image domain where the volumetric data. The image domain is the portion of space defined between the x-ray source XD and the detector D, in which space the region of interest (through which the contrast agent passes) resides during imaging. Thus, the region of interest includes in particular the portion of the vasculature VS to be imaged, with, at times, contrast agent residing therein. Conceptually, the image domain may be thought to be made up of a 3D grid of spatial points ("voxels"). Reconstruction operation R includes computing image values at each of the grid points (voxels), thus obtaining the volumetric data $R(\lambda)$.

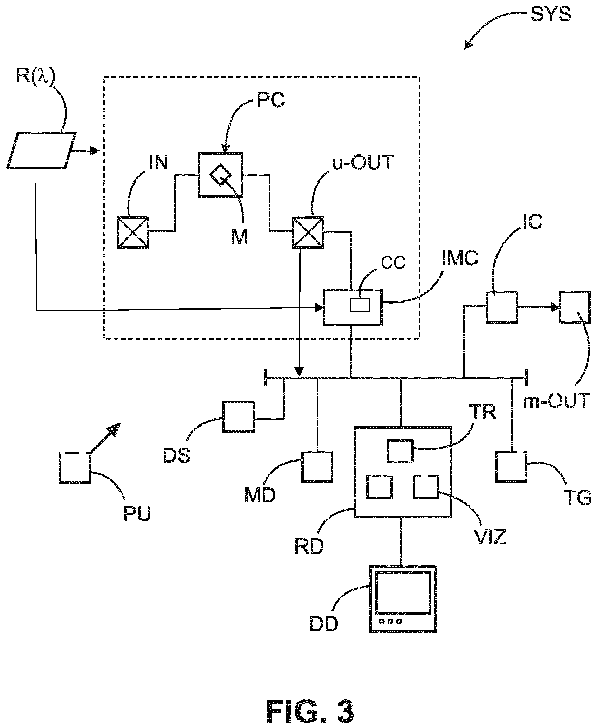

Reference is now made to the block diagram of Fig. 3 which provides more details of components of the system SYS, configured to compute flow increase due to contrast agent presence.

At one or more input ports/interfaces IN, input data including the spectral volumetric data V=R($\lambda$) is received. The output data may comprise a single volume, or a series of volumes acquired over a time period *I* as mentioned above.

A predictor/estimator module PM computes an estimate $\Delta u$ for the flow increase caused by the presence of the contrast agent, based in in the input data. The increase in blood flow $\Delta u$ may be represented in percentage values (*p%*) for example, or by any other suitable quantification. Output data *u-OUT* provided by predictor/estimator module PM may include estimate $\Delta u$ may be output at output port OUT for further processing, storage for later review/processing, etc. Such processing may include operation of a visualizer VIZ for displaying the output data (or data derived from output data) on the display device DD. In particular, such data processing my include processing output data by an in-image measuring component IMC.

The in-image measuring component IMC analyzes voxel data (such as grey values, their contrast distribution in particular due to contrast agent, etc), in the volumetric imagery V and computes therefrom the mentioned flow relates quantities. The said hemodynamicquantities are preferably configures to describe blood flow or flow of the blood-contrast mixture through the vasculature VS of interest.

From such flow related quantities, other appropriate medical quantities of interest may be derived, such as other diagnostic quantities, such as scores for disease presence. The later scores or other diagnostic quantities may be computed by a diagnostic system DS.

The hemodynamic quantities or other flow-related quantities computable by the in-image measuring component IMC may include one or more of: FFR, IFR, CFR, IR, TAG (transluminal attenuation gradient), perfusion scores, and/or others still.. TAG may be determined using average contrast opacification (measured in Hounsfield units for example) in a series of regions of interest along vessel of interest. For example, the ROIs may be evenly spaced, perpendicularly to centerline of the vessel, such as of a coronary artery. TAG procedure may be configured to measure the gradient (rate of fall-off) of contrast opacification along the vessel. For example, TAG may be defined as an average of opacifications and how this average changes along vessel. For example, TAG may be computed as regression coefficients of a line fitting a plot of average such contrast opacification versus distance from a reference point, such as the coronary ostium for example. Such TAG type processing is described by A J Einstein in Journal of the American College of Cardiology, vol. 61, issue 12, 2013, pp 1280-1282.

The increase in flow caused by the presence of the contrast agent (and as estimated by flow-increase predictor PC) may compromise, if not accounted for, the accuracy of the said quantities, if the image measurement component IMC were to compute such quantities on the reconstructed imagery alone as is. Thus, it is proposed herein that the in in-image measuring component IMC uses the estimate for increase in flow as computed by the predictor module PM to so provide an improved (such as more accurate and/or with better intra/inter patient comparability) hemodynamic quantity.

Thus, in-image measuring component IMC computes the hemodynamic quantities (hemodynamic, flow-based, etc) based on the input data (including the image data) and the flow increase estimate. Specifically, it is proposed herein in some embodiments that the in in-image measuring component IMC uses the estimate for increase in flow as computed by the predictor module PM to compensate for such flow increase by correction, normalization or by otherwise modifying an initial measurement by the in-image measuring component IMC to so provide an improved (such as more accurate) hemodynamic quantity. Thus, there may be explicit, distinct such compensation step. This compensation may be handled by the in-image measuring component IMC itself, or by a downstream, internal or external compensator component/service CC. The so improved quantities can then be stored, yet further processed by diagnostic system DS as mentioned above, or displayed, as desired. Alternatively, the flow increase estimate is used by the in-image measuring component IMC already when computing the hemodynamic quantities. Thus, there is no need for initial version quantities that need to be corrected in a second step. The flow estimate is included in the computation of the hemodynamic quantity from the start. Thus, the flow increase is accounted for implicitly within the computation. The operation of accounting for the estimated increased flow when computing hemodynamic quantity $d$, may be also referred herein as a $\Delta u$-compensation/compensating (operation).

$\Delta u$-compensation/compensating may be configured as a linear or nonlinear function C that maps $\Delta u$ (such as percentages) to a corresponding compensating effect $\omega$, such as an offset to be applied to hemodynamic quantity $d$. A threshold-based policy may be used to implement the $\Delta u$-compensation operation. For example, if the estimated flow increase $\Delta u$ is $p\%$ and is in excess of $p_0\%$, the same percentage value $p\%$ (or a suitably scaled version $\beta^* p\%$) may be used to mark down (or-up) an initially computed/measured diagnostic value that was solely based on the spectral image data Vt. For example, a computation of an FFR value may be increased or decreased by $p\%$, if the increase in flow has been found to $p\% > p_0$. The thresholding is not necessary herein in all embodiments, and a floating mark-up or down-compensator policy may be applied instead. Whether the estimated increase $\Delta u$ attracts a related mark-up or mark-down of the image-based hemodynamic quantity $d$, may depend on the type or nature of the hemodynamic quantity $d$, the underlying modelling, and medical rationale etc.

The $\Delta u$- operation (such as correction, normalization, offset mark-down etc) based on the computed flow $\Delta u$ may be done by simple subtraction of the corresponding percentage value, or may be done by forming ratios of initial diagnostic values based on a normalizing/scaling factor computed from the increased flow, etc. The $\Delta u$- operation may take any suitable form where the $\Delta u$ value is suitably combined with (initial) diagnostic value(s) and/or image values of input spectral image $V$ or series thereof, for one or more ROIs. For example, a resting flow velocity measurement ($\Delta u$ formulated as a %-increase) may be corrected in one example embodiment as $d_{corrected} = d_{measured}/(c + \Delta u)$ or similar, or any function thereof. Constant c may be a non-zero constant, such as c=1. As another example, microvascular resistance IMR (again, with $\Delta u$ formulated used as % increase), may be corrected in one example embodiment as

$d_{corrected} = d_{corrected}^* (c + \Delta u)$. In general, the $\Delta u$- operation (such as correction, etc) may thus be formulated as a function of i) the applicable measured value of the hemodynamic quantity $d$, and ii) a factor of $1/(c + \Delta u)$ or $(c + \Delta u)$.

The estimated increased flow may be taken relative to a normal flow expected in the vessel if no contrast agent was present. This normal base line flow can be estimated based on medical knowledge or can be computed for example during ramp up phase or wash-out phase when very little contrast agent is present, but just enough to allow a rough estimate for the normal base-line blood flow. For such a low concentration of contrast agent, the autoregulator response of the vessel caused by the contrast agent's presence is negligible. However, such knowledge of the base line flow is optional, and may not be needed for practicing the principles described herein for obtaining more accurate/reliable image-based diagnostic value $d$.

The flow $\Delta u$ computed herein may be understood as volumetric flow or flow velocity. Volumetric flow, or flux, describes the amount (volume or mass) of contrast agent/blood mixture that passes in unit time through a unit area. Both, volumetric flow and flow velocity may be computed, or only one of the two, as required. The exact form and

expression of $\Delta u$ may depend on the nature of quantity d that one is considering improving. Thus, in some cases volumetric flow may be called for, in some other cases a flow velocity.

The flow increase estimate as provided by the predictor PC may be a single value $\Delta u = u_0$, or may be a time curve $\Delta u(t)$ of such estimates, for example when the input data $V = Vt$ has such a time series character. There may be one (such as exactly one) such estimate $\Delta u(t)$ per frame $V(t)$, or more than one. Yet higher dimensional applications are also envisaged, were there is also a location s dependency for spatial resolution: $\Delta u(t,s)$. Computing such spatio-temporally resolved flow increase compensations are specifically and preferably envisaged in some embodiments. A dependency $\Delta u(s)$ that varies spatially only is also envisaged. In case there is a time series of flow estimates $\Delta u(t)$, multiple such $\Delta u$ values may be used for $\Delta u$-compensating the hemodynamic quantity d, or a single such $\Delta u$ value may be used per hemodynamic quantity d.

For example, one "scan" (set of projection data) can be used to reconstruct such as time series of multiple 3D images Vt for different time points t, e.g. if the CT IA keeps rotating multiple times. Thus, the $\Delta u$ values may be computed at different time points to provide a time resolved curve of flow increase estimates $\Delta u(t)$ as mentioned above. Such a time resolved $\Delta u$-curve may then be used to correct a single hemodynamic quantity describing the whole time range, or multiple such single hemodynamic quantity, one or more per time point t. For example, in perfusion imaging a time-to-peak-contrast may be measured. 3D images Vt for multiple time points before the peak manifests exist. Each 3D image Vt may result in a different, respective flow increase prediction $\Delta u(t)$. And the time-to-peak estimate may be affected by some or all previous flow states. As a further option, and more generally, time dependent flow increase estimates $\Delta u(t)$, together with their matched time dependent hemodynamic quantities d(t), may be used to extrapolate a corrected hemodynamic quantity in a specific flow increase state (e.g. $\Delta u = 0$) that is never actually measured.

Computing TAG as the hemodynamic quantity is one example where the proposed system is used with multiple ROIs (such as the ones described above taken along the vessels) where measurements (obfuscations) are $\Delta u$-compensated.

A vital sign measuring device MD may collect vital sign measurements of the patient such as temperature, blood pressure, etc. These vital sign measurements may be combined with the corrected medical quantities to compute a more advanced hemodynamic model $m(VS) \ni O_j$ of the vasculature VS of interest. In simpler embodiments, the $\Delta u$ values and/or the hemodynamic quantities d derived therefrom may be sufficient to construct such a hemodynamic model.

The estimated increased flow $\Delta u$ data provided by predictor module PM may be used by renderer RD to provide a sensory rendition that can be interpreted or otherwise used/consumed by a human user, such as medical personnel. For example, a transducer TR may be used that provides an alert signal such as visual, auditory, or haptic or otherwise, in case estimated flow increase exceeds a certain threshold at which point the accuracy of the hemodynamic quantities computed by the IMC may be severely compromised and counter-measures may be advisable. For example, the user may choose to wait until the contrast agent concentration has decreased sufficiently so that the autoregulatory response of opening up the vasculature or sections thereof subsides, and to then base the computation of the hemodynamic quantities on volumetric data obtainable at that later time.

Thus, the computed value $\Delta u$ suitably rendered may provide a quality control feature that allows user to better assess accuracy, veracity or reliability of the computed diagnostic values d, based on image information alone. The user may then manually initiate the $\Delta u$-compensation operation, rather than this being done automatically. Even if $\Delta u$-compensation operation is done automatically, a thresholding may still be used as mentioned above to initiate $\Delta u$-compensation operation in respect of d-values only when a certain threshold $p_0$ is exceeded. Thus, the $\Delta u$-values may be used for monitoring purposes, for example to quality control diagnostic value output by in-image measuring component IMC.

Visualizer VIZ may be used by renderer RD to produce a graphics display which can be displayed on the display device DD. Such graphic display may include for example the current imagery and additional data that informs the user of the estimated flow increase $\Delta u$. For example, a bar symbol may be indicated that assumes different colors and/or length in concert with the estimated $\Delta u$ values. Thus, the graphics display may be updated in real time and/or dynamically as new image data is processed to compute corresponding $\Delta u$ values, as these are mapped onto the bar symbol or any other symbol/widget that allows representing the changing values of $\Delta u$. A time curve may be used instead of, or in addition to, such GUI widget to represent changing values.

The hemodynamic quantities as improved/computed based on the $\Delta u$ values may be processed by the diagnostic system DS which may compute other parameters, such as scores for certain diseases for example. Such scores may support or inform downstream therapeutic measures.

The hemodynamic quantities as improved/computed based on the $\Delta u$ data, or indeed the $\Delta u$ data itself, may be supplied to a control interface IC. The control interface IC may provide a control signal or parameter m-OUT to control other medical equipment or processes, in addition or instead to any one or more of the above mentioned systems (diagnostic system DS, transducer TR, etc). For example, control signal or parameter m-OUT may be used to support the ongoing imaging procedure by (re-)setting parameters of the imager IA, and or by controlling the contrast agent delivery apparatus DA (such as a pump), etc.

The predictor module PM may be implemented by using analytical modelling based for example on thresholding, or similar. In some such embodiments, the amounts of contrast agent causing the flow increase, such mass or volume of the contrast agent present, is extracted from the spectral input imagery by a segmentation operation. Thanks to the contrast in the 3D spectral imagery being tuned to the contrast agent material, a precise voxel based thresholding may be done to identify contributions of contrast agent, thus facilitating a good estimation of contrast agent amount. Based on the extracted contrast agent amount, the increase in blood flow caused by the presence of contrast agent can be computed. Thus, extracted contrast agent amount may be mapped to flow increase $\Delta u$ values, such as using a linear function. The linear function maps the extracted amount of contrast agent to the increase in blood flow. Non-linear functions may be used instead. The explicit model function, for example its coefficients and form etc, may be based on medical knowledge. For example, underlying physiological aspects of the autoregulatory response may be modelled in the analytical model function explicitly.

However, such explicit analytical modelling of the spectral image information-vs-flow increase relationship is not necessarily required herein as generic machine learning approaches are also envisaged, instead of, or in addition to, such analytical modelling (hybrid-modelling). Thus, the predictor model PM may be implemented by a trained machine learning model M, trained on previous training data including imagery that may be found in medical databases for example. A regression model, or classifier model may be used. A neural network model may be used.

It is thought that the flow increase is the result of a possibly complex physiological interplay of various factors. As mentioned above, for example the displacement of blood in a vessel for a certain period of time by the contrast agent is causing a kind of "fight and flight" reaction of the body where vasodilation occurs, thus leading to the observed flow increase. However, the precise nature and onset of such vasodilation may differ from patient to patient due to their physiological characteristics such as body mass index, age, sex, patient history etc. Thus, there is assumed to be a latent relationship between contrast agent concentration/blood dilution and the associated flow increase. But this relationship may be complex: for example. The CA-induced vasodilation may not necessarily set in exactly when the maximum amount of contrast agent has accumulated. Instead, there may be a certain delay $\delta$ after maximum CA-concentration, after which the vessel dilates and flow increase. And there is possibly another delay $\delta'$ during which blood is beginning to refill this dilated section of the vessel. Modelling the applicable physiological characteristics and/or the mentioned delay factors $\delta$, $\delta'$ analytically may be challenging. Also, such autoregulatory vessel response may differ from patient to patient. Thus, in order to better account for these physiological effects such as delays $\Delta$, $\Delta'$ and others, in a preferred embodiment the predictor PM uses machine learning ("ML") where such relationship can be modelled using a generic model trained on clinical training data or training data experimentally generated, and/or synthetically generated by simulation, etc. In general, the predictor PM, whether implemented in ML or analytically, is configured to correlate the input spectral imagery with flow changes, such as flow increase, or flow decrease in other embodiments.

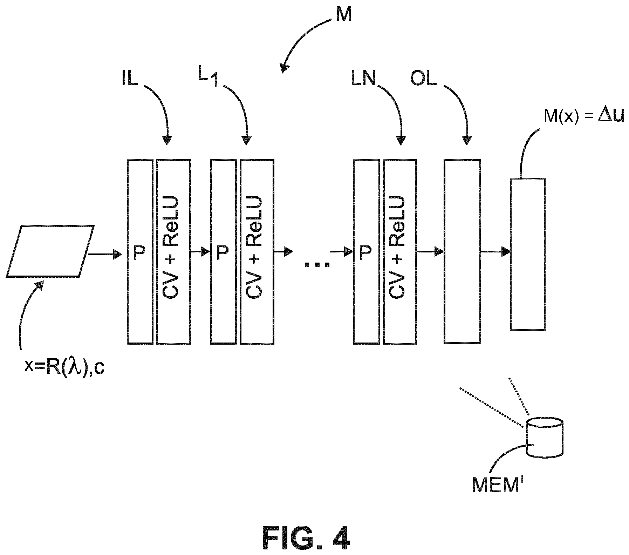

Reference is now made to Fig. 4 which shows a block diagram of a machine learning implementation of the predictor module PM according to one embodiment.

Specifically, the machine learning model M may be arranged as an artificial neural network ("NN"), preferably as an NN of the convolutional type, referred to as "CNN". CNNs are useful in processing spatially correlated data such as image data, the processing of which is envisaged herein as explained earlier.

The network M may comprise a plurality of computational nodes arranged in layers in a cascaded fashion, with data flow proceeding from left to right and thus from layer to layer. The model may thus be of the feedforward type. Recurrent networks are not excluded herein, in particular if input data is a time series of volumes Vt.

The model network M may be said to have a deep architecture because it has more than one hidden layers. In a feed-forward network, the "depth" is the number of hidden layers Lj ($1 \leq j \leq N$) between input layer IL and output layer OL, whilst in recurrent networks the depth is the number of hidden layers, times the number of passes.

The machine learning model includes certain parameters, such as weights of convolution filters CV, that are adjusted in the training phase which will be explained in more detail below. Once trained, that is, once the parameters are suitably adjusted, input x, including input contrasted imagery V, possibly enriched by contextual data c, is input and received at an input layer IL during deployment or testing. The input x is hence either V or (V, $\kappa$). The input data x in deployment or testing propagates through the layers of the model and emerges as the desired result M(X)=$\Delta u$ as the output layer OL. Thus, at output layer OL, the desired result $\Delta u$ is produced. Thus the output may be a regressed into a scalar value $\Delta u$, in some embodiment one such scalar for a given time t and location s.

Thus, the output layer OL may be a regression layer where the input data, including imagery *V* or (*V,c*) is regressed into a single scalar value $\Delta u$, possibly with (s,t) dependence. Alternatively, a classifier layer OL may be used, where the input is classified into $\Delta u$ -value-levels, as such qualitative output may be sufficient in some cases. For example, it may be sufficient in some embodiments for to output to merely indicate into which range/interval the estimated flow increase falls, such as $\Delta u \in [5\%, 10\%]$. In this classifier case output may be a vector with its entries representing the classes of $\Delta u$ -value levels.

In case of a classification result, the output layer OL may be configured as a *softmax*-function layer or as similar computational nodes where feature maps from previous layer(s) are combined into normalized counts to represent the classification probability or scores per class. In a regressional setup, OL may be fully connected layer.

Preferably, some or all of the hidden layers Lj (1≤j≤ N), and optionally the input layer, are convolutional layers, that is, include one or more convolutional filters CV which process an input feature map from an earlier layer into layer-output, sometimes referred to as logits. An optional bias term may be applied by addition for example. An activation layer processes in a non-linear manner the logits into a next generation feature map which is then output and passed as input to the next layer, and so forth. The activation layer may be implemented as a rectified linear unit RELU as shown, or as a *soft-max*-function, a sigmoid-function, *tanh*-function or any other suitable non-linear function. As opposed to a convolution layer, the output layer may be fully connected layer. Hybrid networks, including fully connected and convolutional layers are also envisaged herein.

Additional optional layers may be used in CNN model M, such as drop-out layer, pooling layers P, and other. The pooling layers reduce dimension of output whilst drop-out layer sever connections between nodes from different layers.

The trained model may be stored in one or more data storages MEM'.

Preferably, to achieve good throughput, the computing device PU includes one or more processors (CPU) that support parallel computing, such as those of multi-core design. In one embodiment, GPU(s) (graphical processing units) are used.

However, NN models M are not the only ones envisaged: support vector machines (SVM), decision trees, random forests, linear regression or others still may be used with benefit in some cases. However, because of the spatially correlated nature of input imagery *V*, NN type models have been found to yield good results. Recurrent models such as LSTM are beneficial with time series data.

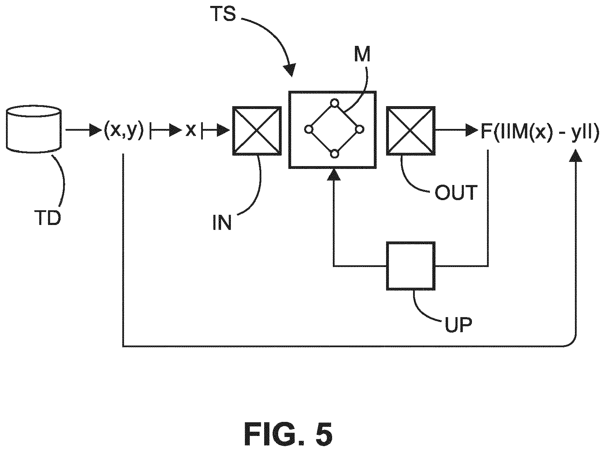

Referring now to Fig. 5, this shows a training system TS that may be used to train such a model M based on training data (*x,y*) as may be held in a training data memory TD. Training data (*x,y*) can be procured from existing medical data such as may be found in a medical data bases, such a PACS, HIS or other database or storage system. Such training data may include historic image data or other health records of patients that have undergone similar examinations in which contrasted imagery of a related ROI was acquired. The historic imagery may be reviewed by a human expert, for example. The expert may review the contrasted imagery of historic patients and may select historic imagery that corresponds, according to their medical knowledge, to a certain flow increase or contrast agent concentration. The human expert may review the patient health record for notes on the respective patient to come to their conclusion. Thus, the human expert may assign a respective score *y* (or label) to different respective samples of training input imagery x. Score *y* ε [*a.b*], on a predefined scale [*a,b*]. Score *y* represents an estimate of the respective flow increase associated by the expert with the respective input image x. information on contrast agent concentration may be used by user to estimate flow increase from their medical experience and expert knowledge.

Experimental options to obtain labels *y* are also envisaged, such as based on phantoms. Also, the training data could be built up over time in interventions where a flow measurement is taken and this is recorded. General relations may be available from prior invasive angiography and could be translated and used for training on CT.

Respective (historic) context data κ, such as historic flow related measurement or remarks, etc may be used to assist the user when assigning their score y. The context κ may be found in the health record of historic patients. In some cases, the health record context *κ* may be used to infer an indication for the flow increase. Specifically, medical records associated with historic imagery may be harvested either by the human expert, or by an automatic text analyzer tool (such as an NLP pipeline or string matcher tool, such as grep or other) to extract the corresponding flow related state as this information may be mentioned in the respective reports or records referring to a specific image. In this way, the collection process can be fully or partially automated, thus allowing to procure suitably labelled training data construct more quickly. The context κ may also include patient bio-characteristics such as sex, weight, height, BMI, etc of patient. Such context data may be processed together with the image training data input to improve learning.

Training as administered by training system TS is the process of adapting parameters of the model based on the training data. An explicit model is not necessarily required as in some examples it is the training data itself that constitutes the model, such as in clustering techniques or k-nearest neighbors. Etc. In explicit modelling, such as in NN-based approaches and many others, the model may include as system of model functions/computational nodes, with their input and/or output it least partially interconnected. The model functions or nodes are associated with parameters θ which are adapted in training. The model functions may include the convolution operators and/or weights of the non-linear units such as a RELU, mentioned above at Fig. 4 in connection with NN-type models. In the NN-case, the parameters θ may include the weights of convolution kernels of operators CV and/or of the non-linear units.

The parameterized model may be formally written as $M^\theta$. The parameter adaptation may be implemented by a numerical optimization procedure. The optimization may be iterative. An objective function f may be used to guide or control the optimization procedure. The parameters are adapted or updated by updater UP so that the objective function is improved. The input training data $x^i$ is applied to the model. The model responds to produce training data output M($x^i$) = $y^i$. The objective function is mapping from parameters space into a set of numbers. The objective function *f* measures a combined deviation between the training data outputs $y^1$ and the respective targets $y^1$. Parameters are iteratively adjusted to that the combined deviation decreases until a user or designer pre-set stopping condition is satisfied. The objective function may use a distance measure ‖ . ‖ to quantify the deviation.

In some embodiments, but not all, the combined deviation may be implemented as a sum over some or all residues based on the training data instances/pairs $(x^i, y^j)^i$, and the optimization problem in terms of the objective function may be formulated as:

$$argmin_{\theta} f = \sum_k \| M^{\theta}(x_k), y_k \| \qquad (1)$$

In the setup (1), the optimization is formulated as a minimization of a cost function $f$, but is not limiting herein, as the dual formulation of maximizing a utility function may be used instead. Summation is over training data instances $i$.

The cost function $f$ may be pixel/voxel-based, such as the L1 or L2-norm cost function. For example in least squares or similar approaches, the (squared) Euclidean-distance-type cost function in (1) may be used for the above mentioned regression task. When configuring the model as a classifier, the summation in (1) is formulated instead as one of cross-entropy or Kullback-Leiber divergence or similar. Huber cost function may be used.

The exact functional composition of the updater UP depends on the optimization procedure implemented. For example. An optimization scheme such as backward/forward propagation or other gradient based methods may be used to adapt the parameters $\theta$ of the model M so as to decrease the combined residue for all or a subset $(x_k, y_k)$ of training pairs from the full training data set. Such subsets are sometime referred to as batches, and the optimization may proceed batchwise until all of the training data set is exhausted, or until a predefined number of training data instances have been processed.

Training may be a one-off operation, or may be repeated once new training data becomes available.

Optionally, one or more batch normalization operators ("BN", not shown) may be used. The batch normalization operators may be integrated into the model M, for example coupled to one or more of the convolutional operator CV in a layer. BN operators allow mitigating vanishing gradient effects, the gradual reduction of gradient magnitude in the repeated forward and backward passes experienced during gradient-based learning algorithms in the learning phase of the model M The batch normalization operators BN may be used in training, but may also be used in deployment.

The training system as shown in Fig. 5 can be considered for all learning schemes, in particular supervised schemes. Unsupervised learning schemes may also be envisaged herein in alternative embodiments. GPUs may be used to implement the training system TS.

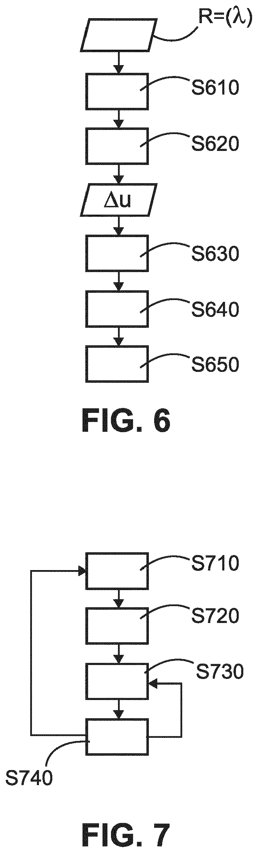

Fig. 6 shows a flow chart of a computer implemented method of estimating increase in blood flow caused by the presence of contrast agent due to auto-regulatory response by the vessel under consideration, and in which said contrast agent is present. The computed increase in blood flow/flux can be used to obtain more accurate diagnostic values such as hemodynamic quantities, or other such quantities that describe or relate to blood flow.

At step S610, volumetric spectral image data is received. The volumetric image data V = R(λ) is based on tomographic data reconstructed from projection data λ acquired by a spectral imaging apparatus ("CTA" or angiographic CT (computed tomography) ). The projection data is acquired whilst at least part of the contrast agent is present at least at times in the vasculature of interest (ROI) in the field of view. The projection data or the volumetric data is processed by a spectral processing algorithm to obtain spectral imagery such as a contrast-only-image data. In such spectral tomographic imagery as is mainly envisaged herein for input data, contrast is pre-dominantly, or exclusively, a function of contrast agent amount. Contributive attenuative effects of intervening structures and/or other materials as projected through by the acquiring X-ray beam are largely eliminated. The notation "$V$" is used herein to designate such image volume obtained after application of such spectral processing algorithm. The input spectral image volume $V$ may include series of such volumes, and there may be more than one ROIs.

At step S620, based on the input spectral volumetric data, the increase in blood flow caused by the present of the contrast agent is computed.

At step S630 this estimated flow increase $\Delta u$ is output as output data. The output data $\Delta u$ may be a single flow increase value, or a time series of such values. The output data may be supplied as percentages, or as flux values, velocity values, etc, as needed. The estimated flow increase $\Delta u$ may relate globally to the ROI as a whole, or may relate locally to a subset of the ROI. The output may spatially resolved to relate to plural ROIs.

At step S640 a hemodynamic quantity $d$, such as a hemodynamic quantity, is computed based on the spectral volumetric input imagery.

At step S650 the computed quantity is compensated for the effect of flow increase such as by correction, normalization, or by other modification, based on the increased flow $\Delta u$ as computed at step S620. Thus, in embodiments of the "explicit" type, an initial version d is computed at step S640, and this is then improved by using the increased flow $\Delta u$ as per step S630. In other embodiments of the implicit type, steps S640 and S650 may be combined instead into a single processing step. If the $\Delta u$-compensation step S650 is applied separately from the step S640 of computing the diagnostic value (rather than be integrated), the order of step S620,S630 and S640 may be reversed.

The $\Delta u$-compensation step S650, whether explicit or implicit, may be applied according to an unconditional policy automatically and/or at all times, or only when a certain condition is met, such as when the estimated flow increase exceeds a threshold. Other conditions than such thresholding may be formulated instead.

Thus, the hemodynamic quantities obtained herein are of better quality, such as more reliable, more accurate and afford better cross-patient comparison as opposed to computing the quantity without the $\Delta u$-compensation step S650. The proposed approach based on $\Delta u$-compensation step S650 allows eliminating, or at least reducing, interfering effects caused by the increase in flow/flux.

The corrected diagnostic value(s) and/or indeed the $\Delta u$ data itself may be provided for processing, such as for computing a score that represents presence of disease, or the value may be stored in storage, displayed, may be used for control purposes, or may be otherwise processed as required, etc.

Some of the above steps are now described in more detail.

For example, the predicting/estimating the flow increase $\Delta u$ at step S630 may be done analytically and/or using machine learning.

In an analytical+ML hybrid setup of step S630, the following sub-steps may be executed:

processing the image data to segment for coronaries or other vessels of interest, and optionally perfusion regions; analyzing image values at voxels, such as HU value, to estimate the blood iodine concentration in the segmented areas. The said analyzing may include thresholding; and

estimating the increase in blood flow $\Delta u$ in segmented areas that is caused by the diluted blood/presence of contrast agent. This can be done directly by estimation of dilution for example. For example, a linear model function may be used to relate contrast agent mass/volume/dilution/concentration to flow increase. A unity proportionality factor $\beta=1$ may be used for example and for simplicity. In such a model M, a p% iodine concentration would lead to a p% increased flow. Such model can be based on a regression model trained on a clinical training data set sourced from medical databases. Non-linear models are also envisaged.

The above hybrid step may be complemented by incorporating hemodynamic modeling of the coronary system. Examples of such hemodynamic modeling may include a lumped resistor model (LRM). In such LRMs, vessels may be modeled as a network of resistor elements, and boundary conditions may be used to model any one or both of the pumping heart, and the microvasculature with its autoregulation. This allows to better take local and temporal variations of the particular vessel system into account. Again, linear type hemodynamic modeling is envisaged herein, but so are non-linear approaches.

However, step S630 may be implemented purely analytically, as described above, or purely in ML. In the latter case, no explicit segmentation or other extraction may be required: the ML model may be trained end-to-end to map from image data V directly to output that represents the desired $\Delta u$ estimate(s).

The estimating step S630 of flow increase caused autoregulation of vessel due to contrast agent presence may further be supported by live vital signs of the patient. E.g. blood pressure and heart rate, etc. Thus, the input may further include such vitals sign parameters as contextual data $\kappa$. Such contextual data $\kappa$ may be useful in particular for ML.

In order to achieve more accurate temporal information of contrast agent concentrations and related flow increase, a series of partial scan reconstructions from the CT data may be performed and used as input rather than a single volume $V$ (although such is also envisaged herein). Using a time series of volumetric spectral data such as a series of partial reconstructions may be useful for normalizing perfusion analysis over longer time scales for example.

The proposed method and system can also be applied when determining functional parameters to quantify peripheral vascular disease.

Reference is now made to Fig. 7 which shows a flow chart of a method of training a machine learning model as may be used in step S620 above.

At step S710 training data is received, for example in the form of pairs $(x_k,y_k)$. Each pair includes the training input $x_k$ and the associated target $y_k$. $x_k$, as defined above.

At step S720, the training input $x_k$ is applied to an initialized machine learning model M to produce a training output.

A deviation, or residue, of the training output $M(x_k)$ from the associated target $y_k$ is quantified by a cost function F at step S730. One or more parameters of the model are adapted at step S740 in one or more iterations in an inner loop to improve the cost function. For instance, the model parameters are adapted to decrease residues as measured by the cost function. The parameters include in particular weights of the convolutional operators, in case a convolutional model M is used.

The training method then returns in an outer loop to step S710 where the next pair of training data is fed in. In step S740, the parameters of the model are adapted so that the aggregated residues of all pairs considered are decreased, in particular minimized. The cost function quantifies the aggregated residues. Forward- backward propagation or similar gradient-based techniques may be used in the inner loop. Instead of looping over individual pairs at step S710, looping is over stets of training data pair, or batches, and these are feed in a processed at once in step S720, and S730.

Examples for gradient-based optimizations may include gradient descent, stochastic gradient. conjugate gradients, Maximum likelihood methods, EM-maximization, Gauss-Newton, and others. Approaches other than gradient-based ones are also envisaged, such as Nelder-Mead, Bayesian optimization, simulated annealing, genetic algorithms, Monte-Carlo methods, and others still.

More generally, the parameters of the model M are adjusted to improve objective function $F$ which is either a cost function or a utility function. In embodiments, the cost function is configured to the measure the aggregated residues. In embodiments the aggregation of residues is implemented by summation over all or some residues for all pairs considered. In particular, in preferred embodiments the outer summation proceeds in batches (subset of training instances), whose summed residues are considered all at once when adjusting the parameters in the inner loop. The outer loop then proceeds to the next batch and so for until the requisite number of training data instances have been processed.

It will be understood that the above embodiments may be modified: instead of predicting/estimating flow increase, a decrease of flow may be estimated instead. Such may be caused by some types of contrast agent, and this effect equally interferes with the computing of standardized diagnostic values $d$ as desired herein. For example, some contrast agents may cause spasms in the microvascular muscle cells. However, the estimating of flow increase as described above is preferred herein.

The components of system SYS may be implemented as one or more software modules, run on one or more general-purpose processing units PU such as a workstation associated with the imager IA, or on a server computer associated with a group of imagers.

Alternatively, some or all components of system SYS may be arranged in hardware such as a suitably programmed microcontroller or microprocessor, such an FPGA (field-programmable-gate-array) or as a hardwired IC chip, an application specific integrated circuitry (ASIC), integrated into the imaging system IA. In a further embodiment still, the system SYS may be implemented in both, partly in software and partly in hardware.

The different components of system SYS may be implemented on a single data processing unit PU. Alternatively, some or more components are implemented on different processing units PU, possibly remotely arranged in a distributed architecture and connectable in a suitable communication network such as in a cloud setting or client-server setup, etc.

One or more features described herein can be configured or implemented as or with circuitry encoded within a computer-readable medium, and/or combinations thereof. Circuitry may include discrete and/or integrated circuitry, a system-on-a-chip (SOC), and combinations thereof, a machine, a computer system, a processor and memory, a computer program.

In another exemplary embodiment of the present invention, a computer program or a computer program element is provided that is characterized by being adapted to execute the method steps of the method according to one of the preceding embodiments, on an appropriate system.

The computer program element might therefore be stored on a computer unit, which might also be part of an embodiment of the present invention. This computing unit may be adapted to perform or induce a performing of the steps of the method described above. Moreover, it may be adapted to operate the components of the above-described apparatus. The computing unit can be adapted to operate automatically and/or to execute the orders of a user. A computer program may be loaded into a working memory of a data processor. The data processor may thus be equipped to carry out the method of the invention.

This exemplary embodiment of the invention covers both, a computer program that right from the beginning uses the invention and a computer program that by means of an up-date turns an existing program into a program that uses the invention.

Further on, the computer program element might be able to provide all necessary steps to fulfill the procedure of an exemplary embodiment of the method as described above.

According to a further exemplary embodiment of the present invention, a computer readable medium, such as a CD-ROM, is presented wherein the computer readable medium has a computer program element stored on it which computer program element is described by the preceding section.

A computer program may be stored and/or distributed on a suitable medium (in particular, but not necessarily, a non-transitory medium), such as an optical storage medium or a solid-state medium supplied together with or as part of other hardware, but may also be distributed in other forms, such as via the internet or other wired or wireless telecommunication systems.

However, the computer program may also be presented over a network like the World Wide Web and can be downloaded into the working memory of a data processor from such a network. According to a further exemplary embodiment of the present invention, a medium for making a computer program element available for downloading is provided, which computer program element is arranged to perform a method according to one of the previously described embodiments of the invention.

It has to be noted that embodiments of the invention are described with reference to different subject matters. In particular, some embodiments are described with reference to method type claims whereas other embodiments are described with reference to the device type claims. However, a person skilled in the art will gather from the above and the following description that, unless otherwise notified, in addition to any combination of features belonging to one type of subject matter also any combination between features relating to different subject matters is considered to be disclosed with this application. However, all features can be combined providing synergetic effects that are more than the simple summation of the features.

While the invention has been illustrated and described in detail in the drawings and foregoing description, such illustration and description are to be considered illustrative or exemplary and not restrictive. The invention is not limited to the disclosed embodiments. Other variations to the disclosed embodiments can be understood and effected by those skilled in the art in practicing a claimed invention, from a study of the drawings, the disclosure, and the dependent claims.

In the claims, the word "comprising" does not exclude other elements or steps, and the indefinite article "a" or "an" does not exclude a plurality. A single processor or other unit may fulfill the functions of several items re-cited in the claims. The mere fact that certain measures are re-cited in mutually different dependent claims does not indicate that a combination of these measures cannot be used to advantage. Any reference signs in the claims should not be construed as limiting the scope. Such reference signs may be comprised of numbers, of letters or any of alphanumeric combination.

**Claims**

1. System for medical image processing, comprising

   an input interface (IN) through which is receivable, when the system is in use, input data including spectral input imagery of at least a part of a vascular system (V) of a patient (PAT), the spectral input imagery based on data reconstructable from projection data acquired by a spectral imaging apparatus of the tomographic type, with presence of contrast agent in the part of the vascular system;
   a predictor module (PM) configured to predict, based on the input imagery, an increase or decrease in flow caused by presence of contrast agent; and
   an output interface (u-OUT) for providing output data indicative of the predicted flow increase or decrease.

2. System, of claim 1, comprising an in-image measurement component (IMC) configured to measure a medical quantity of interest based on the input imagery and the said output data.

3. System of claim 2, wherein the in-image measurement component (IMC) to combine information from the input imagery with information based on the said output data.

4. System of claim 3, wherein the combining by the in-image measurement component (IMC) includes correcting or normalizing, based on the said output data, an initial measurement of the medical quantity of interest by the in-image assessment component (IMC) based on the input imagery.

5. System of any one of the previous claims, wherein the medical quantity of interest includes a hemodynamic quantity, such as any one or more of: perfusion score, fractional flow reserve, FFR, instantaneous flow ratio iFR, coronary flow reserve, CFR, index of microvascular resistance, IMR, and transluminal attenuation gradient, TAG.

6. System of any one of the previous systems, including a renderer (RD) configured to provide a sensory indication based on the output data for consumption by a user, wherein the renderer (RD) includes any one or more of: i) a visualizer (VIZ) configured to cause displaying on a display device (DD) of the output data, ii) a transducer (TR) configured to generate an alert signal in relation to the output data.

7. System of any one of the previous claims, including a control interface (IC) configured to issue a control signal based on the output data to control medical equipment.

8. System of any one of the previous claims, wherein the increase or decrease in blood flow is caused by an autoregulation response of the vascular system due to the presence of contrast agent.

9. System of any one of the previous claims, wherein the predictor module (PM) is based on a trained machine learning model (M).

10. System of any one of the previous claims, wherein spectral imaging apparatus is configured for computed tomography angiography.

**11.** An arrangement (MIA) including a system as per any one of the previous claims, and the imaging apparatus.

**12.** Method of medical image processing, comprising:-

receiving (S610) input data including spectral input imagery of at least a part of a vascular system (VS) of a patient (PAT), the spectral input imagery based on data reconstructable from projection data acquired by a spectral imaging apparatus of the tomographic type, with presence of contrast agent in the part of the vascular system;

estimating (S620), based on the input imagery, an increase or decrease in flow caused by presence of contrast agent; and

providing (S630) output data indicative of the predicted flow increase or decrease.

**13.** Method of training the machine leaning model as per claim 9 based on training data.

**14.** A computer program element, which, when being executed by at least one processing unit, is adapted to cause the processing unit (PU) to perform the method as per any one of claims 12,13.

**15.** At least one computer readable medium having stored thereon the program element of claim 14, or having stored thereon the machine learning model as per claim 9 or 13.

FIG. 1

**FIG. 2**

**FIG. 3**

FIG. 4

**FIG. 5**

$R=(\lambda)$

S610

S620

$\Delta u$

S630

S640

S650

# FIG. 6

S710

S720

S730

S740

# FIG. 7

Europäisches
Patentamt

European
Patent Office

Office européen
des brevets

## EUROPEAN SEARCH REPORT

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 2015/297161 A1 (GRASS MICHAEL [DE] ET AL) 22 October 2015 (2015-10-22) | 1-8, 10-12, 14,15 | INV. A61B6/00 |
| Y | * abstract * <br> * figures 1-5 * <br> * paragraph [0002] – paragraph [0081] * <br> ----- | 9,13 | |
| X | RACHID FAHMI ET AL: "Quantitative myocardial perfusion imaging in a porcine ischemia model using a prototype spectral detector CT system", PHYSICS IN MEDICINE AND BIOLOGY, INSTITUTE OF PHYSICS PUBLISHING, BRISTOL GB, vol. 61, no. 6, 4 March 2016 (2016-03-04), pages 2407-2431, XP020301392, ISSN: 0031-9155, DOI: 10.1088/0031-9155/61/6/2407 [retrieved on 2016-03-04] * abstract * * figures 1-11 * * Sections 1 – 4 * ----- | 1,12,14, 15 | |
| | | | **TECHNICAL FIELDS SEARCHED (IPC)** |
| Y | EP 3 649 955 A1 (KONINKLIJKE PHILIPS NV [NL]) 13 May 2020 (2020-05-13) * abstract * * figures 1-10 * * paragraph [0002] – paragraph [0213] * ----- | 9,13 | A61B |

The present search report has been drawn up for all claims

| Place of search | Date of completion of the search | Examiner |
|---|---|---|
| Munich | 24 February 2023 | Moehrs, Sascha |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

## ANNEX TO THE EUROPEAN SEARCH REPORT
## ON EUROPEAN PATENT APPLICATION NO.

EP 22 19 9939

This annex lists the patent family members relating to the patent documents cited in the above-mentioned European search report.
The members are as contained in the European Patent Office EDP file on
The European Patent Office is in no way liable for these particulars which are merely given for the purpose of information.

24-02-2023

| Patent document cited in search report | | Publication date | Patent family member(s) | | Publication date |
|---|---|---|---|---|---|
| US 2015297161 | A1 | 22-10-2015 | BR 112015013248 | A2 | 11-07-2017 |
| | | | CN 104871211 | A | 26-08-2015 |
| | | | EP 2932469 | A1 | 21-10-2015 |
| | | | JP 5953438 | B2 | 20-07-2016 |
| | | | JP 2016504084 | A | 12-02-2016 |
| | | | RU 2015128005 | A | 19-01-2017 |
| | | | US 2015297161 | A1 | 22-10-2015 |
| | | | WO 2014091339 | A1 | 19-06-2014 |
| EP 3649955 | A1 | 13-05-2020 | CN 112969412 | A | 15-06-2021 |
| | | | EP 3649955 | A1 | 13-05-2020 |
| | | | EP 3876842 | A1 | 15-09-2021 |
| | | | JP 2022509016 | A | 20-01-2022 |
| | | | US 2021386389 | A1 | 16-12-2021 |
| | | | WO 2020094596 | A1 | 14-05-2020 |

EPO FORM P0459

For more details about this annex : see Official Journal of the European Patent Office, No. 12/82

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Non-patent literature cited in the description**

- **T. M. MITCHELL.** Machine Learning. McGraw-Hill, 1997, 2, , 6

- **A J EINSTEIN.** *Journal of the American College of Cardiology,* 2013, vol. 61 (12), 1280-1282